# EUROPEAN PATENT SPECIFICATION

(11) **EP 4 188 474 B1**
(45) Date of publication and mention of the grant of the patent: **22.04.2026**
(21) Application number: 20775728.7
(22) Date of filing: 29.07.2020
(51) Int. Cl.: A61M 1/16, A61M 1/28

(54) **PERITONEAL DIALYSIS CONCENTRATE POUCHES**
PERITONEALDIALYSEKONZENTRATBEUTEL
POCHES DE CONCENTRÉ DE DIALYSE PÉRITONÉALE

(43) Date of publication of application: 07.06.2023
(73) Proprietor: Bellco S.r.l., 41037 Mirandola (IT)
(72) Inventor: VISCIANO, Angela, 41037 MIRANDOLA (IT); PAGATINI, Guilherme, 41037 MIRANDOLA (IT); VESPERINI, Genni, 41037 MIRANDOLA (IT); LUPOTTI, Marco, 41037 MIRANDOLA (IT)
(74) Representative: Greaves Brewster LLP
(86) International application number: PCT/IT2020/000059
(87) International publication number: WO 2022/024148

(56) References cited:
- EP-A1- 3 466 460
- EP-A1- 3 903 851
- US-A1- 2004 217 057
- US-A1- 2010 154 647

## Description

### FIELD

The disclosure relates to concentrate pouches for use in peritoneal dialysis. The pouches can initially contain a solid, liquid, or aqueous solution. Water can be added to the concentrate pouches to generate concentrates containing constituent parts of a peritoneal dialysis fluid for use in peritoneal dialysis therapy. The concentrate pouches can induce a venturi effect to assist dissolution of concentrates.

### BACKGROUND

The known peritoneal dialysis fluid generation systems and methods sometimes generate peritoneal dialysis fluid from premixed bags or ready-to-use bags of liquid concentrated forms of peritoneal dialysis fluid. The known systems and methods require a large amount of storage space to contain the liquid peritoneal dialysis fluid, and are generally incapable of generating peritoneal dialysis fluid from solid materials in single use containers. The known systems and methods cannot generate peritoneal dialysis fluid with solutes in a range of concentrations that can be varied independent of any other solutes. Although some known systems and methods rely on mechanical mixing or agitation to dissolve dry powder, the systems often fail to provide sufficient or adequate mixing. One known system uses a diffuser configured as a series of spaced bars extending radially from a central pipe on an interior floor of a compartment containing a dry powder concentrate. The diffuser is intended to create a turbulent flow to dissolve the dry powder in the compartment. Still other systems and methods use various tube arrangement to dissolve dry powder. In one arrangement, fluid exits from a top of a tube and flows downward to dissolve powder in a container. However, the known systems often fail to completely or adequately dissolve dry powders when generating a concentrate solution within certain conditions such as temperature and pressure. The known systems are also often expensive, not disposable, and cannot be customized. The known systems can also require long periods of time to sufficiently or adequately dissolve powders or solid materials. Known systems employing pouches to prepare dialysate are disclosed in e.g. US2004/0217057 A1 or EP 3 466 460 A1.

As such, there is a need for systems and methods that can sufficiently dissolve dry powder concentrate or solid materials. The need extends to an effective system for adequately and timely dissolving dry powders in a container and/or sufficiently mixing liquid concentrates. The need includes dissolving dry powders within certain time periods. The need includes varying the concentrations of solutes in the peritoneal dialysis fluid independently of any other solutes by having a modular system of concentrate containers containing either dry power or liquid concentrates. The need includes a system of dry powder peritoneal dialysis constituent components that can be customized to fit a patient's individual prescription or therapy goals. There is a further need for systems and methods that allow generation and use of concentrates for peritoneal dialysis from solid or liquid materials. The need extends to an easy to use system for preparing a peritoneal dialysis fluid in an in-home setting. The need extends to a system for generating peritoneal dialysis fluid that minimizes shipping volumes and storage space for constituent components.

### SUMMARY OF THE INVENTION

The invention is defined by the features of independent claims 1 and 17. The first aspect of the invention relates to a concentrate pouch. In any embodiment, the concentrate pouch can include a fluid inlet positioned at a bottom portion of the pouch wherein the fluid inlet includes a venturi tube entering an interior of the concentrate pouch; the venturi tube can have a first section distal to the interior portion of the concentrate pouch, the first section having a first diameter, a second section distal to the first section and proximal to the interior portion of the concentrate pouch, the second section having a second diameter smaller than the first diameter, and a third section distal to the second section and extending inwardly into the interior of the concentrate pouch, the third section having a third diameter larger than the second diameter, and having an suction port at or about a floor of the interior of the concentrate pouch, the suction port exposing an interior of the venturi tube to the interior of the concentrate pouch.

In any embodiment, the concentrate pouch can contain a solid material.

In any embodiment, wherein the first diameter of the first section or the third diameter of the third section is about four times the second diameter of the second section.

In any embodiment, the concentrate pouch can be constructed from a flexible material.

In any embodiment, the concentrate pouch can be constructed from a flexible material and can be a stand up pouch

In any embodiment, wherein the floor of the concentrate pouch inwardly tapers towards the fluid inlet.

In any embodiment, the venturi tube can extend upwardly from a bottom of the concentrate pouch towards a top of the concentrate pouch.

The features disclosed as being part of the first aspect of the invention can be in the first aspect of the invention, either alone or in combination, or follow any arrangement or permutation of any one or more of the described elements. Similarly, any features disclosed as being part of the second or third aspects of the invention can be in the first aspect of the invention, either alone or in combination, or follow any arrangement or permutation of any one or more of the described elements.

The second aspect of the invention is drawn to a system. In any embodiment, the system can include one or more of the concentrate pouches of the first aspect of the invention fluidly connected to a peritoneal dialysis fluid generation system; and a control system programmed to pump water into the one or more concentrate pouches to generate a concentrate; and to pump a concentrate from the one or more concentrate pouches into the peritoneal dialysis fluid generation flow path and into a mixing container.

In any embodiment, the system can include a water source fluidly connectable to the one or more concentrate pouches.

In any embodiment, the one or more concentrate pouches can include a concentrate pouch containing dextrose, icodextrin or polydextrin, a concentrate pouch containing magnesium, calcium and sodium lactate, and a concentrate pouch containing sodium bicarbonate and sodium chloride.

In any embodiment, the one or more concentrate pouches can include a concentrate pouch containing dextrose, icodextrin or polydextrin, a concentrate pouch containing magnesium and calcium, and a concentrate pouch containing sodium bicarbonate, sodium chloride, and sodium lactate.

In any embodiment, the one or more concentrate pouches can include a concentrate pouch containing dextrose, icodextrin or polydextrin, a concentrate pouch containing magnesium, calcium, and sodium lactate, and a concentrate pouch containing sodium chloride.

In any embodiment, the one or more concentrate pouches can include a concentrate pouch containing dextrose, icodextrin or polydextrin, a concentrate pouch containing magnesium and calcium, and a concentrate pouch containing sodium chloride and sodium lactate.

In any embodiment, at least one of the concentrate pouches can initially contain a solid substance.

In any embodiment, at least one of the concentrate pouches can initially contain a liquid or aqueous substance.

In any embodiment, the control system can be programmed to pump an amount of water into the one or more concentrate pouches to completely dissolve materials within the one or more concentrate pouches.

In any embodiment, at least one of the one or more concentrate pouches can be a stand up pouch.

The features disclosed as being part of the second aspect of the invention can be in the second aspect of the invention, either alone or in combination, or follow any arrangement or permutation of any one or more of the described elements. Similarly, any features disclosed as being part of the first or third aspects of the invention can be in the second aspect of the invention, either alone or in combination, or follow any arrangement or permutation of any one or more of the described elements.

The third aspect of the invention is drawn to a method. In any embodiment, the method can include the steps of pumping water into one or more concentrate pouches of the first aspect of the invention; dissolving a material within the one or more concentrate pouches to generate a concentrate in each of the one or more concentrate pouches; and pumping the concentrate out of each of the one or more concentrate pouches.

In any embodiment, the method can include the step of pumping the concentrate from each concentrate pouch to a mixing container.

In any embodiment, the one or more concentrate pouches can include a concentrate pouch containing dextrose, icodextrin or polydextrin, a concentrate pouch containing magnesium, calcium and sodium lactate, and a concentrate pouch containing sodium bicarbonate and sodium chloride.

In any embodiment, the one or more concentrate pouches can include a concentrate pouch containing dextrose, icodextrin, or polydextrin, a concentrate pouch containing magnesium and calcium, and a concentrate pouch containing sodium bicarbonate, sodium chloride, and sodium lactate.

In any embodiment, the one or more concentrate pouches can include a concentrate pouch containing dextrose, icodextrin or polydextrin, a concentrate pouch containing magnesium, calcium, and sodium lactate, and a concentrate pouch containing sodium chloride.

In any embodiment, the one or more concentrate pouches can include a concentrate pouch containing dextrose, icodextrin, or polydextrin, a concentrate pouch containing magnesium and calcium, and a concentrate pouch containing sodium chloride and sodium lactate.

The features disclosed as being part of the third aspect of the invention can be in any other part of the third aspect of the invention, either alone or in combination, or follow any arrangement or permutation of any one or more of the described elements. Similarly, any features disclosed as being part of the first or second aspect of the invention can be in the third aspect of the invention, either alone or in combination, or follow any arrangement or permutation of any one or more of the described elements.

### BRIEF DESCRIPTION OF THE DRAWINGS

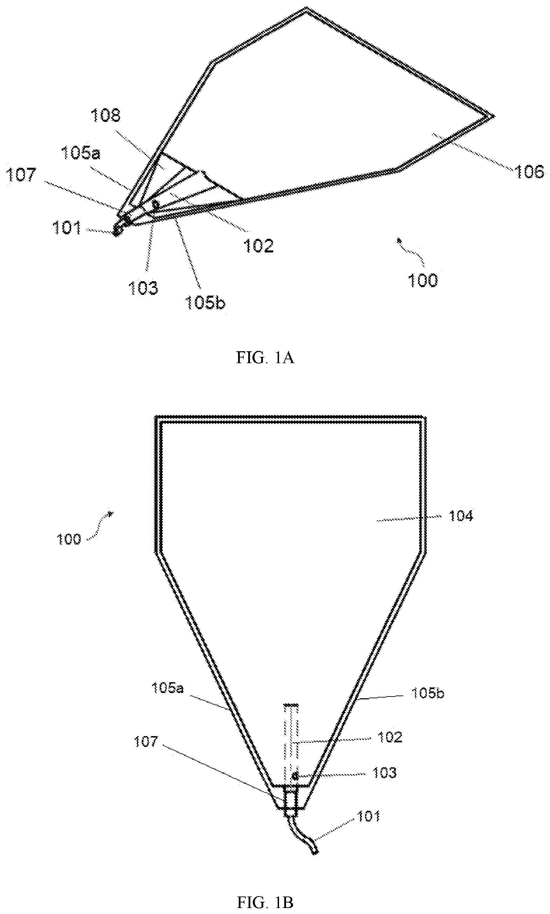

FIG.'s 1A-B illustrate a concentrate pouch. FIG. 1A is a perspective view of a cutaway showing one side of the concentrate pouch. FIG. 1B is an exterior side view of the concentrate pouch.
FIG. 2 illustrates a concentrate pouch standing up alone having a threaded connector and a protective cap.
FIG. 3 illustrates a concentrate pouch having a rigid integrated connector and a removable cap flap.
FIG. 4 illustrates a concentrate pouch having a rigid connector and a peelable bottom portion.
FIG. 5 illustrates multiple concentrate pouches configuration used to generate a peritoneal dialysate.
FIG.'s 6A-F illustrate close up views of a concentrate pouch and integrated Venturi.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The term "at least" refers to a possibility of one or more elements may be present. For example, a container containing at least magnesium provides for the possibility of containing one or more other components such as calcium.

The terms "aqueous," "aqueous substance," or an "aqueous solution" refer to a solution of a solute in water.

The term "bottom portion" refers to a surface or section nearest to a lowest point of the component or section when in an upright orientation.

The term "calcium," as used herein, can refer to any source of calcium, such as calcium chloride.

The term "completely dissolve" refers to the process of generating a solution from a solute in which essentially no solute remains undissolved.

The term "comprising" includes, but is not limited to, whatever follows the word "comprising." Use of the term indicates the listed elements are required or mandatory but that other elements are optional and may be present.

A "concentrate" refers to a solution of one or more solutes in water. The concentrate can have a solute concentration greater than that to be used in dialysis.

The term "concentrate pouch" refers to a container that can contains at any point in time any one or more of a solid such as powder, fluid, solutions, and any combination thereof. At a desired point, the concentrate pouch can contain a concentrate fluid to be formed or contained therein. The pouch can be made of any suitable material and can initially contain dry, wet, or a combination of dry and wet materials where the contents and state of the materials inside the container can change at any time.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "constriction" refers to any portion of a feature that is reduced in size, in reference to a compared feature such as for a tube, pipe, or flow path. For example, the constriction can have a smaller diameter relative in reference to a rounded tube or pipe. The constriction can mean any smaller opening relative to a section to which the constriction is being compared to, and is not limited to any specific relative size or shape. For example, a constriction can be flattened tube that has a smaller cross-sectional area of the flow path as compared to an adjoining tubular pipe that has a larger cross-sectional area of the flow path.

The term "constructed from" refers to a type of material that makes up a component.

The terms "contain" or "containing" refer to a material held within a component or container. The term contain is open ended and does not prevent the inclusion of other components being included within the same component.

The terms "control," "controlling," or "controls" can refer to the ability of one component to direct the actions of a second component.

A "control system" can be a device that monitors and affects the operational conditions of a given system. The operational conditions are typically referred to as output variables of the system wherein the output variables can be affected by adjusting certain input variables. The control system can any number or combination of processors, controllers, software, and computers.

The term "dextrose" refers to a simple sugar compound with a chemical formula C₆H₁₂O₆. Dextrose is the dextrorotatory form of glucose, and in certain embodiments, the terms dextrose and glucose can be used interchangeably.

The term "diameter" refers to a distance from one side of a circle to an opposite side of the circle through the center of the circle.

The term "dissolve" refers to the process of dissolution or more broadly, the generation of a solution from a solvent and solute.

The term "distal" refers to a section or component positioned away from a point of attachment or origin.

The term "downstream" refers to a position of a first component in a flow path relative to a second component wherein fluid will pass by the second component prior to the first component during normal operation. The first component can be said to be "downstream" of the second component, while the second component is "upstream" of the first component.

The term "exposing" in context of an suction port means allowing either side of the suction port having access to the other side of the opening or suction port.

The terms "extend" or "extending" refer to a portion of a component directed or situation in a specified direction or orientation.

The terms "first," "second," and "third," and the like, refer to separate and distinct features. For example, one or more sections can be identified as a 'first section," "second section," and "third section." Alternatively, one or more diameters can be identified as a 'first diameter," "second diameter," and "third diameter."

The term "flexible material" refers to a material that can change shape in response to applied pressure.

A "fluid path" of "fluid line" can refer to a tubing or conduit through which a fluid, gas, or a combination thereof can pass. The fluid path or line can also contain air during different modes of operation such as cleaning or purging of a path or line.

The term "fluidly connectable" refers to the ability to provide passage of fluid, gas, or combinations thereof, from one point to another point. The ability to provide such passage can be any mechanical connection, fastening, or forming between two points to permit the flow of fluid, gas, or combinations thereof. The two points can be within or between any one or more of compartments, modules, systems, components, all of any type. Notably, the components that are fluidly connectable, need not be a part of a structure. For example, an outlet "fluidly connectable" to a container does not require the container pump, but merely that the outlet has the features necessary for fluid connection to the container.

The term "fluidly connected" refers to a particular state or configuration of one or more components such that fluid, gas, or combination thereof, can flow from one point to another point. The connection state can also include an optional unconnected state or configuration, such that the two points are disconnected from each other to discontinue flow. It will be further understood that the two "fluidly connectable" points, as defined above, can form a "fluidly connected" state. The two points can be within or between any one or more of compartments, modules, systems, components, and rechargers, all of any type.

An "fluid inlet" is a portion of a component through which gas, fluid, and combinations thereof can enter or exit the component. Although the term inlet generally refers to an opening for entry of gas, fluid, and combinations thereof, the inlet can sometimes provide a means for exiting or exhausting the gas, fluid, and combinations thereof. For example, during a priming, cleaning, or disinfection, the inlet can be used to remove gas, fluid, and combinations thereof through the inlet. Also, during operation, the inlet can remove gas, fluid, and combinations thereof.

The terms "forming," "forms," and the like refers to any type of shape or structure of an object or feature.

The phrase "four times" refers to multiplying a value by four.

The phrase "generate a concentrate" or "generating a concentrate" refer to a process or steps for dissolving a solid or liquid material, or diluting a solution, to form a concentrated solution.

The term "icodextrin" refers to a water-soluble glucose polymer.

The terms "initial" or "initially" in reference to feature or object refers to a beginning or starting condition or state of the feature. For example, a "concentrate container initially containing a solid substance" refers to the container starting with a solid substance wherein a "subsequent" state or condition of the concentrate container can change, such as where a solid material is reconstituted by the addition of water. Similarly, the term "subsequent" refers to a following state or condition of the feature or object.

The term "interior" refers to an inside portion or section of an object or component. The term can also refer to a surface such as an interior wall of a tube, pouch, or container.

The term "inwardly tapering" describes a shape of a container or component that has a decreasing diameter, inclination, or size towards a location or point in a specified direction.

The term "liquid," "liquid substance," or a "liquid material" refer to a material in the liquid phase of matter.

The term "magnesium," as used herein, can refer to any source of magnesium, such as magnesium chloride.

A "mixing container" is a container into which one or more fluid streams can be flowed to generate a mixture. The mixing can occur by passive turbulence or active means such as an agitator.

An "opening" is a portion of a component that through which fluid or gas can enter or exit the component.

"Peritoneal dialysis fluid" is a dialysis solution to be used in peritoneal dialysis having specified parameters for purity and sterility. Peritoneal dialysate is not the same as dialysate used in hemodialysis.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

A "peritoneal dialysis fluid generation flow path" is a fluid flow path that can receive fluids, concentrates, and/or water for the generation of a peritoneal dialysis fluid.

The term "positioned" refers to a component, feature, or process step connected to or in relation to the feature being referred to, or the relative location with respect thereto. The contact can be physical, fluid, temporal, or electrical and is intended to be used in the broadest reasonable sense.

The term "polydextrin" refers to any polymer formed from dextrose or glucose monomers.

The term "pouch" can refer to a flexible bag of any size and shape fabricated from a suitable material to hold any one of the concentrates of the invention.

The term "programmed" is to be interpreted in the broadest sense and can mean any series of instructions that cause a processor or a control system of any type including software to perform certain steps or to execute an algorithm of any type. For example, a processor can be programmed to execute an algorithm. Alternatively, a computer can be programmed to execute a series of functions of steps.

The term "proximal" refers to a section or component positioned nearest or adjacent to a point of attachment or origin.

The terms "pumping," "pumped," or to "pump" refer to moving or flowing a fluid using a pump of any type known to those of ordinary skill in the art.

The terms "pumping fluid" or to "pump fluid" refer to moving a fluid, gas, or a combination thereof through a flow path of any type with a pressure. The pressure can be generated by a pump of any type.

"Sodium bicarbonate" refers to NaHCO₃, either in solution or solid form.

"Sodium chloride" refers to NaCl, either in solution or solid form.

"Sodium lactate" refers to C₃H₅NaO₃, either in solution or solid form.

The terms "solid," "solid substance," or a "solid material" refer to a material in the solid phase of matter, and can include crystalline, powdered, or any other form of solid material.

The term "stand up pouch" refers to a concentrate pouch having a shape or components that will generally maintain the concentrate pouch in an erect position without support from other components.

The term "suction port" refers to an aperture or opening in a component or feature, commonly a tube or flow path, through which any material, fluid, gas, or combinations thereof can flow. Generally, flow is directed inwards through the suction port using any motive force such as a vacuum. However, the suction port is not constrained to inwardly directed flow wherein material, gas, or combinations thereof can flow outwards from the suction port under certain conditions.

The term "tube" refers to a conduit through which material, fluid, gas, or combinations thereof can flow.

The term "upwardly" refers to a direction from a bottom of a component towards the top of the component when the component is positioned for normal use.

The term "upright" refers to an orientation of a component that is the same vertical orientation as intended for use.

The term "venturi" as used in a "venturi tube," "venturi tube," "venturi effect," "venturi system," and the like, generally refer to a reduction in fluid pressure that results when a fluid flows through a constricted section of a flow path such as a tube.

The term "water source" refers to a source from which any one of potable, purified, or sterile water can be obtained.

### Peritoneal Dialysis Pouches

FIG. 1A is a perspective cut-away of an interior side **106** of a peritoneal dialysis concentrate pouch **100.** The concentrate pouch **100** can initially contain a solid or concentrated material of any type. The concentrate pouch **100** can contain any solid, liquid, aqueous solution, and combinations thereof in any state of mixing, partial dissolution, or complete dissolution. Upon dissolution and reconstitution of a solid such as powder, the solid contents of the pouch (not shown) can dissolve from a solid powder to a liquid solution, for example, by the addition of water. Fluid can be pumped into the concentrate pouch **100** through fluid tube **102,** which can be fluidly connectable to a fluid line **101.** The fluid line **101** can connect to a peritoneal dialysis fluid generation system (not shown) for generation of peritoneal dialysis fluid from the concentrate in concentrate pouch **100.** The fluid tube **102** can extend upwardly into the concentrate pouch **100** and can include an integrated venturi feature of any type or configuration to induce a low-pressure zone after a constriction in the venturi. The low-pressure zone can be used to draw up material to dissolve solid material and form a solution within the concentrate pouch **104.**

The integrated venturi feature or system of the invention can have at least one suction port **103** in fluid tube **102,** as well as a constriction **107** in fluid tube **102** (not shown in FIG.'s 1A-B) to create a low-pressure zone due to the venturi effect. The constriction **107** inside the venturi system of fluid tube **102** increases flow velocity and reduces pressure after the constriction **107.** As fluid flow exits the constriction **107,** a vacuum is created by the pressure drop as the fluid expands in a downstream section of fluid tube **102.** The reduced pressure after the constriction **107** creates a vacuum such that material adjacent to a suction hole **103** is drawn into the fluid tube **102.** The suction port **103** can be positioned at or about a floor of the interior of the concentrate pouch **100** to expose an interior of the fluid tube **102** to the interior of the concentrate pouch 100. The suction created by the venturi effect can draws in any material such as dry powder into suction port **103.** The negative pressure at suction port **103** can draw in any dry powder or concentrate into the primary flow along the fluid tube **102.** At the end of the fluid tube **102,** a mixture of liquid and concentrate can appear without any separate mechanical agitation. The suction port **103** can be downstream of the constriction **107** at any suitable position on fluid tube **102.** Additional suction ports anywhere from one or more suctions ports can be included in fluid tube **102.** In one embodiment, up to six or more suction ports can be used. The suction ports can be exposed to an interior space inside the concentrate pouch **100.** As such, material inside concentrate pouch **100** can be drawn in through the suction port **103** positioned in the low-pressure zone after the constriction **107** in the venturi. The fluid tube **102** can have a constant outer diameter while having diameters of varying size inside the venturi system of the fluid tube **102.** The material can then travel upwardly for any length of the fluid tube **102** along with any fluid being flowed into the concentrate pouch **100.** The combined fluid/material mixture can exit at a top of the fluid tube **102** inside the concentrate pouch **101.**

FIG. 1B is a side view of an exterior side **104** of the concentrate pouch **100.** The fluid tube **102** is positioned at or near a bottom portion of the concentrate pouch **100** in an upright position. The fluid tube **102** enters the interior of the concentrate pouch **100** at the bottom portion of the concentrate pouch **100.** The fluid tube **102** has a first section distal to the interior portion of the concentrate pouch **100** such that fluid flowing through the fluid tube **102** encounters the first section with a first diameter. Fluid then flows into a second section defining the constriction **107** having a smaller diameter than the first section. Fluid then flows into a third section having a third diameter larger than the second section. The first diameter is larger than the constriction **107** in the second section or the diameter in the third section. Similarly, the diameter of the third section is larger than one or both of the first and second section. The static pressure in the first section of the fluid tube **102** can be higher than at the second section downstream of the first section wherein any fluid speed in the first section is lower than in the second section due to a greater diameter in the first section relative to the second section.

The second section defining the constriction **107** is proximal to the interior portion of the concentrate pouch **100** with respect to the third section of the fluid tube **102.** The constriction **107** induces a higher fluid velocity but lower pressure. The diameter of the constriction **107** in the venturi system of the second section can be any suitable diameter relative to the first diameter sufficient to create a desired venturi effect or vacuum force in the third section. The third section of fluid tube **102** is distal to the second section and extends into the concentrate pouch **100** for any length In one embodiment, the fluid tube **102** extends to a top of the concentrate pouch **100.** In another embodiment, the fluid tube **102** terminates at or near the suction port **103.** As fluid exits the second section, a pressure drop is created in the third section and creates a vacuum that can suck up material adjacent to suction port **103.** The third section has a third diameter larger than the constriction **107.** The third section can create a turbulent flow and contain internal ridges or screw features (not shown) positioned along the length of an interior side of the fluid tube **102** to encourage mixture of materials suctioned into the fluid tube **102.** By drawing material through the suction port **103** positioned at or near the bottom portion of the concentrate pouch **100,** the materials can be mixed in the flowing fluid and more quickly dissolved. The mixture can then exit directly from the fluid tube **102** inside the concentrate pouch **100.** The fluid/material mixture can then be flowed back through the suction port **103.** Alternatively, the flow can reverse and mixed fluid, material, and/or solution can exit in the reverse direction through fluid tube **102.** One or more pumps can reverse to provide the necessary motive force to draw the concentrate solution back through fluid tube **102.**

The fluid tube **102** can extend any distance into the concentrate pouch **100.** In certain embodiments, the fluid tube **102** can extend to a maximum of 10 mm into concentrate pouch **100.** However, the fluid tube **102** can extend farther or shorter into the concentrate pouch **100,** including distances greater than 10 mm. Dissolution can be unaffected over a range of sizes. As disclosed herein, any number of suction ports can be positioned in the low pressure region to draw in material into the flow path inside fluid tube **102.** In certain embodiments, the inner walls of the pouch body **104** can include an inclination **105a** and/or inclination **105b.** In general, inclination **105a** and inclination **105b** can be an inwardly tapering of the inner walls of the pouch body **104** towards the fluid tube **102** at the bottom portion of the pouch body **104.** The inclination **105a** and inclination **105b** can act to collect all materials including sold material, powders, partially dissolved concentrate, and/or fully dissolved concentrate at the bottom of the concentrate pouch **100.** The collected materials and or solutions can be sucked through suction port **103** and dissolved or further mixed. Further, the inclination **105a** and inclination **105b** can help to avoid the creation of no-flow zones within the concentrate pouch **100.** In certain embodiments, the inclination **105** of the inner walls can be between 20°-60°. In other embodiments, the bottom portion can be formed into a funnel to direct materials towards the suction port **103.** Vertical ribs positioned on the interior side of the funnel can encourage materials and solutions to flow towards the bottom of the pouch body **104.**

The fluid tube **102** can connect to fluid line **101** of a peritoneal dialysis generation system by any means known in the art. For example, the bottom of fluid tube **102** can include a capped and threaded portion that can mate with a threaded portion on a peritoneal dialysis system. Alternatively, the fluid tube **102** can include a rigid connector for connection to the peritoneal dialysis generation system. A flap (not shown in FIG.'s 1A-B) can cover the rigid connector prior to connection to separate the contents of the concentrate pouch **100** from the outside environment. Alternatively, a peelable layer can be placed on the bottom of fluid tube **102** that can be removed prior to connection. The peelable layer can be formed from a polyolefin film or metal foil. The material can be made from any long-lasting, non-reactive material suitable for storage, sterility, and transport. The peelable layer can be made from any of a polymer selected from at least one of LLDPE (Linear Low Density Polyethylene), LDPE (Low Density Polyethylene) and HDPE (High Density Polyethylene), ethylene-vinyl acetate copolymer, ethylene-methyl acrylate copolymer and HDPE or any suitable material. The materials should form a consistent hermetic seal for leak protection and preserve the materials contained inside. The materials should provide tamper-evident protection but be sufficiently easy to open or peel.

The concentrate pouch **100** can be constructed from a flexible or rigid material, as desired. In certain embodiments, the concentrate pouch **100** can be made from mono or multilayer film material, including polypropylene-polyethylene or polypropylene-polyethylene-polyamide for multilayer polyolefin-based films; a monolayer polyolefin film such as polypropylene or polyethylene, or vinyl based films such as PVC or EVA. Alternatively, any other suitable material known to those of skill in the art can be used. In general, the materials should be non-reactive to the materials contained inside the concentrate pouch **100.** The concentrate pouch **100** can be made from a flexible material formed from two pieces where the pouch side **106** can be welded to another pouch side (not shown). The pouches and/or containers can also be semi-flexible, semi-rigid, or rigid. The pouches and/or containers can be fabricated from any suitable process known to those of ordinary skill. One of ordinary skill will understand that the pouch can be made from any suitable material depending on any desired manufacturing, usage, and size constraints.

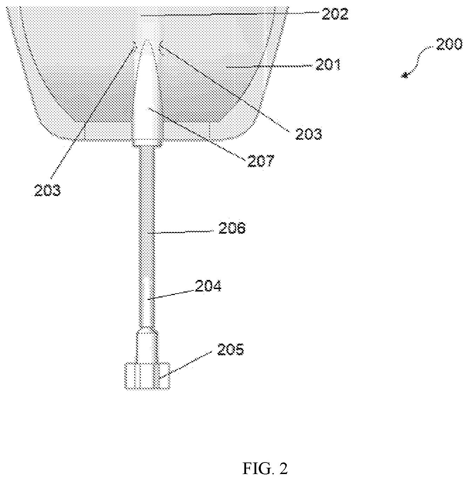

FIG. 2 illustrates a single concentrate pouch **200** standing upright with a protective cap **205.** The protective cap **205** can be connected to an external section **206** of fluid tube **202** at section **204.** The protective cap **205** can be connected by any means suitable for connecting and disconnecting the protective cap **205** such as a thread, snap-connection, or luer connection. The protective cap **205** can be used when transporting the concentrate pouch **200.** In an alternative embodiment, the protective cap **205** can be part of a peritoneal dialysis fluid generation system (not shown) wherein a user first unscrews a cap (not shown) from the section **204,** which is threaded, and then screws the threaded protective cap **205** to section **204** to form a fluid connection from the peritoneal dialysate fluid generation system to the concentrate pouch **205.** In other embodiments, the protective cap **205** can be simply removed when the concentrate pouch **200** is connected to a fluid line or fluid generation system. Alternatively, the protective cap **205** can be integral to the external section **206.** The protective cap **205** can be sterile or non-sterile depending on the desired connection requirement. The external section **206** of the fluid tube **202** can direct fluid to and from the concentrate pouch **200.** The external section **206** of the fluid tube **202** can be connected to venturi **207** of the fluid tube **202.** The venturi **207** can generate a pressure drop at or around one or more suction port **203.** The suction port **203** can be positioned anywhere fluid tube **202** where a pressure drop results from the venturi effect. The suction port **203** can be positioned at or near the bottom so that material falls to at or nearest the lowest point of the concentrate pouch **200** in an upright position. As described, the venturi can speed dissolution of solids inside the pouch body **201** and to help generate a homogenous solution. Although shown as substantially flat in FIG. 2, the interior walls of the concentrate pouch body **201** can include an inclination, similar to that shown in FIG.'s 1A-B. Alternatively, the concentrate body **201** can be formed into a funnel-shape.

In certain embodiments, the concentrate pouch **200** can be a stand up pouch. For example, protective cap **205** can have a substantially flat bottom and a large enough diameter to allow the concentrate pouch **200** to remain erect or upright without any additional components supporting the concentrate pouch **200.** Alternative components, such as supports (not shown) can also be included to allow the concentrate pouch **200** to stand upright.

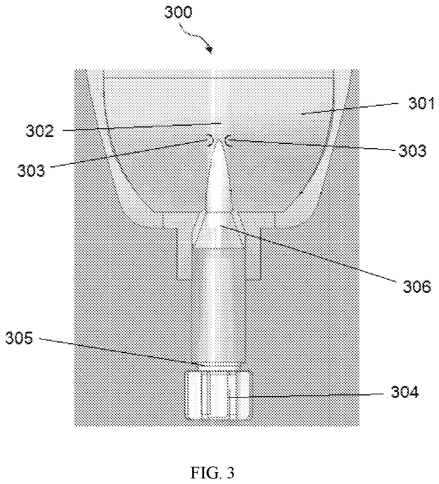

FIG. 3 illustrates a single concentrate pouch **300** standing upright having a rigid integrated connector **300** with a removable cap **304.** The removable cap **304** can initially cover connector **305** on the concentrate pouch **300.** For use, a user can remove or open removable cap **304** and connect the pouch connectors **305** to a connector on a peritoneal dialysis fluid generation system (not shown). A fluid line of the peritoneal dialysis fluid generation system can connect to fluid tube **302** of the concentrate pouch **300** via connector **305.** Fluid can then flow into a constriction **306** that creates a high velocity, low pressure area. As fluid exits constriction **306** a pressure drop can create a vacuum. The fluid tube **302** can include at least one suction port **303** after fluid exits constriction **306** to speed dissolution of solids inside the pouch body **301** and help generate a homogenous solution. Although shown as two suction ports **303** in FIG. 3, the fluid tube **302** can include more suction ports, including between 1 and 6 or more suction ports. Although shown as substantially flat, the pouch body **301** can include an inclination at the bottom of the pouch body **301** to improve dissolution of solids and generate a homogeneous concentrate solution. The bottom of the pouch body **301** can also be formed into a conical or funnel shape. The fluid tube **302** can extend for any length in the interior of the pouch body **301.**

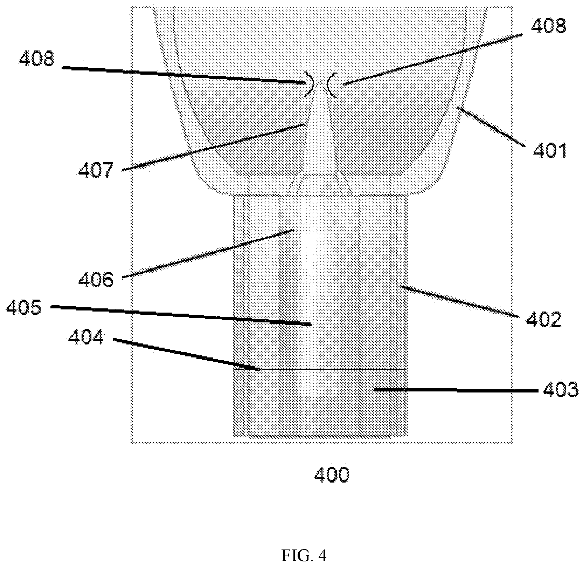

FIG. 4 illustrates a single concentrate pouch **400** positioned upright having a rigid fluid connector **405** positioned at the bottom of the concentrate pouch **400** and a peelable bottom portion **403** covering an open end of the pouch **400** The concentrate pouch **400** can include an external layer **402,** the bottom of which is a peelable portion **403,** which can be peeled away at a line **404.** Once peeled, the rigid fluid connector **405** can connect to a complimentary connector (not shown) on a peritoneal dialysis fluid generation system to provide a fluid communication. A fluid line (not shown) of the peritoneal dialysis fluid generation system can be fluidly connected to a tube path **406** of the rigid fluid connector **405.** The tube path **406** can have a construction 407 to create an area of high fluid velocity and low pressure. Fluid exiting the constriction **407** can experience a pressure drop. One or more suction ports **408** can vacuum any material or fluid into the fluid path to speed dissolution of solids inside the pouch body **401.** The pouch body **401** can include an inclination at the bottom of the pouch body **401** to improve dissolution of solids. Alternatively, the bottom of the pouch body **401** can be in a funnel or conical form to encourage material to collect at the bottom of the pouch body **401.**

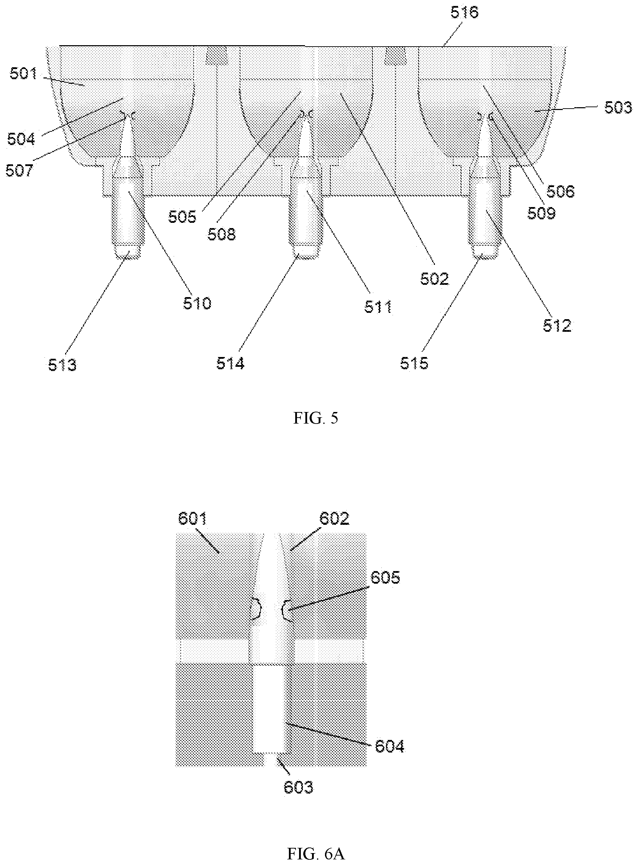

FIG. 5 illustrates a system using concentrate pouch **501,** concentrate pouch **502,** and concentrate pouch **503** in an upright orientation. Although shown as three concentrate pouches in FIG. 5, one of skill in the art will understand that any number of concentrate pouches can be used depending on the needs of the system and user. For example, a system requiring two different components that must be dissolved separately can contain a series of two pouches, while a system requiring four or more different components that must be dissolved separately can contain a series of four or more pouches. As illustrated in FIG. 5, concentrate pouch **501,** concentrate pouch **502,** and concentrate pouch **503** can be connected together by welding or by any suitable structure or component such as a rigid frame. The pouches can be formed into a horizontal line **516** or any suitable configuration. The concentrate pouch **501,** concentrate pouch **502,** and concentrate pouch **503** can be connected together directly at the sides or any other portion of the concentrate pouch **501,** concentrate pouch **502,** and concentrate pouch **503.** The one or more concentrate pouches of the invention need not be connected, and can instead be separate non-connected pouches.

Each of the concentrate pouch **501,** concentrate pouch **502,** and concentrate pouch **503** can include a fluid tube **504,** fluid tube **505,** and fluid **506,** respectively, to draw fluid into and out of the respective concentrate pouch **501,** concentrate pouch **502,** and concentrate pouch **503.** As described, each fluid tube **504,** fluid tube **505,** and fluid **506** can include an integrated venturi system and one or more suction port **507,** suction port **508,** and suction port **509,** respectively. The concentrate pouch **501,** concentrate pouch **502,** and concentrate pouch **503** can connect to a peritoneal dialysis fluid generation system by any means, including the methods illustrated in FIG.'s 2-4. In certain embodiments, the external portion **510,** external portion **511,** and external portion **512** of the respective fluid tube **504,** fluid tube **505,** and fluid tube **506** can fluidly connect to fluid lines of the peritoneal dialysis generation system through connector **513,** connector **514,** and connector **515,** respectively.

Water can be added to each of the concentrate pouch **501,** concentrate pouch **502,** and concentrate pouch **503,** dissolving solid or liquid material or diluting a liquid material or solution in the pouch bodies. In certain embodiments, a water purification system can be included in the peritoneal dialysis fluid generation system to purify an incoming water stream prior to pumping the water into the concentrate pouch **501,** concentrate pouch **502,** and concentrate pouch **503.** Alternatively, the concentrate pouch **501,** concentrate pouch **502,** and concentrate pouch **503** can be fluidly connected to a purified water source. Once the material inside each of concentrate pouch **501,** concentrate pouch **502,** and concentrate pouch **503** is dissolved or diluted to a specified concentration, concentrate can be pumped out of each of the concentrate pouch **501,** concentrate pouch **502,** and concentrate pouch **503** to a mixing container, and integrated peritoneal dialysis cycler, or to a storage reservoir. The amounts of each concentrate added from each of the concentrate pouch **501,** concentrate pouch **502,** and concentrate pouch **503** can be varied to arrive at a predetermined dialysate formulation. A control system (not shown) can be included to control the generation of peritoneal dialysis fluid from the concentrates. The control system can be programmed to pump a specified amount of water into each of concentrate pouch **501,** concentrate pouch **502,** and concentrate pouch **503** to generate concentrates having specified concentrations. The control system can then operate one or more valves and pumps (not shown) to pump the concentrates out of the concentrate pouches and into the peritoneal dialysis fluid generation system, generating a peritoneal dialysis fluid having specified concentrations of each of the components.

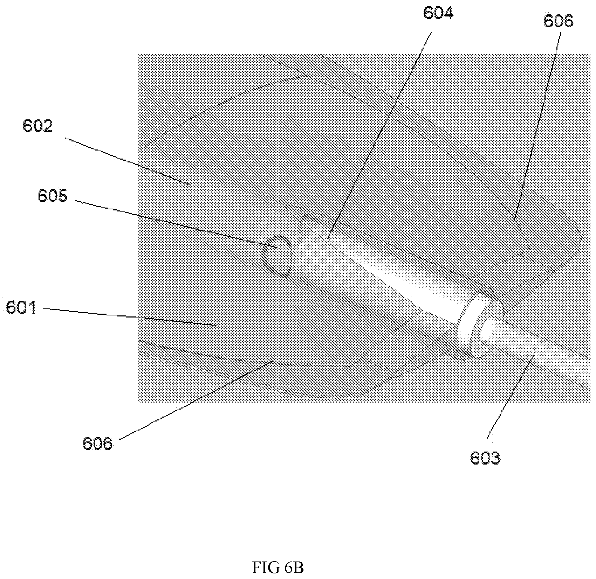

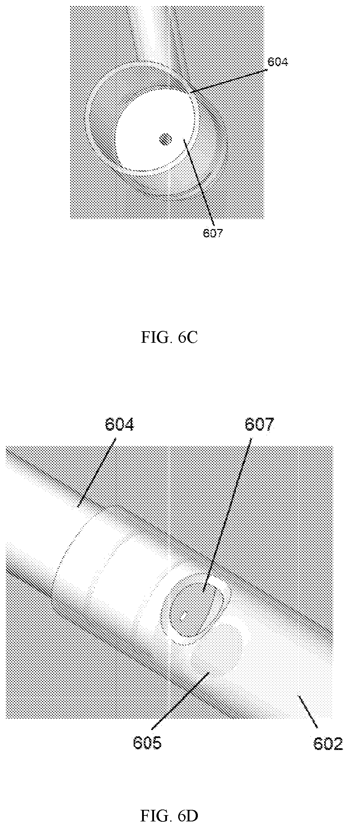

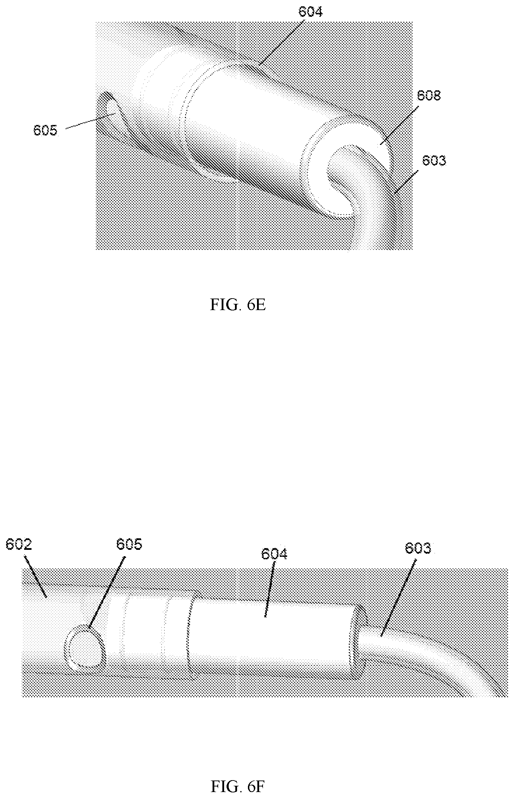

FIG.6A shows one non-limiting embodiment of close up view of an interior **601** of a concentrate pouch. The interior **601** of the concentrate pouch is in fluid connection to an interior section **602** of a fluid tube. The exterior section **603** of the fluid tube is outside the concentrate pouch. The exterior section **603** can connect to a fluid line of a peritoneal dialysis fluid generation system. The fluid tube can include a venturi section **604** and one or more suction port **605** downstream of the venturi section **604** inside the interior **601** of the concentrate pouch. FIG. 6B shows the venturi section **604** that can contain a constriction (not shown) wherein the exterior section **603** of the fluid tube can be inserted into the bottom of the venturi section **604.** The interior section **602** of the fluid tube can be inserted into the top of the venturi section **604.** The interior portion **602** and the exterior section **603** of the fluid tube can be formed as part of, and made integral, or inserted into venturi section **604.** Any suitable mechanism or arrangement can be used to connect the interior portion **602** and the exterior section **603** to the venturi section **604.** FIG. 6C show a cutaway, top perspective of the interior section **602** of the fluid tube. The interior cross section **607** can have a larger area as defined by circumference of the top of the venturi body **604** as compared to a fluid exiting the venturi section **604** into the interior portion **602** of the fluid tube. FIG. 6D is a side perspective showing one or more suction port **605** downstream of venturi section **604** in interior section **602** of the fluid tube. The interior cross section **607** has an area of low pressure which can create a vacuum to suck in material and fluids into the fluid tube via suction port **605.** FIG. 6E shows a first diameter **608** of the venturi section **604.** Fluid enters the bottom of the venturi section **604** via the exterior section **603** of the fluid tube. Once exiting the venturi section **604,** fluid can be mixed with material and fluids being sucked in the fluid tube via suction port **605.**
The smaller and larger diameters of the venturi system can be any size capable of generating the required fluid pressure differential. In certain embodiments, the diameter of the venturi section **604** can be up to 5 mm. The fluid tube can have a diameter of between 2-6 mm around the suction ports **605.** One or more suction ports **605** can be included, in certain embodiments the number of suction ports **605,** and can range between two and six. The diameter of a constriction inside venturi section **604** can range from about 1 mm, while the larger diameter can be about 4 mm. The diameter of the suction port **605** can be around 5 mm. One of ordinary skill in the art can adjust the diameters of any section of the venturi and the diameters of the suction ports to obtain a suitable dissolution under a set of specified conditions. Moreover, the length of the tube inside the interior of the concentrate pouch can be any suitable length. One set of non-limiting parameters can include flow speed, dissolution time, pressure, and temperature.

Experiments were conducted and show that without a venturi system, sodium bicarbonate in a concentrate pouch did not dissolve in less than 10 minutes at a flow rate of 60 ml/min into the concentrate pouch. However, using a venturi system and a water inlet temperature of 48°C, the sodium bicarbonate was completely dissolved in 2.24 minutes providing a final solution temperature of 45°C. Table 1 shows results for a similar experiment using dextrose in a concentrate pouch without a venturi system. Although dissolution tests are influenced by the final concentration, temperature, and other parameters, the experiments show that the venturi system does speed dissolution under the conditions tested.

**Table 1**

| Time to Dissolve/Mix (min) | Temperature after Dissolution/Mixing (°C) |
|---|---|
| 130 | 24.5 |
| 45 | 34.6 |
| 30 | 40 |

As shown in Table 1, the dextrose took longer to dissolve or mix at lower temperatures. Without a venturi system, at 24.5 °C, the dextrose took 130 minutes to dissolve, at 34.6°C the dextrose took 45 minutes to dissolve and at 40°C the dextrose took 30 minutes to dissolve. However, when using a venturi system, the dextrose was dissolved in 4 minutes at 49°C. The venturi system significantly reduced the time of dissolution for all possible powders tested.

As described, peritoneal dialysis fluid can be generated using one or more concentrate pouches. Table 2 provides non-limiting examples of combinations and sizes of pouches that can be used. One of skill in the art will understand that alternative arrangements of materials across one or more concentrate pouches can be included, and Table 2 is provided for illustrative purposes only.

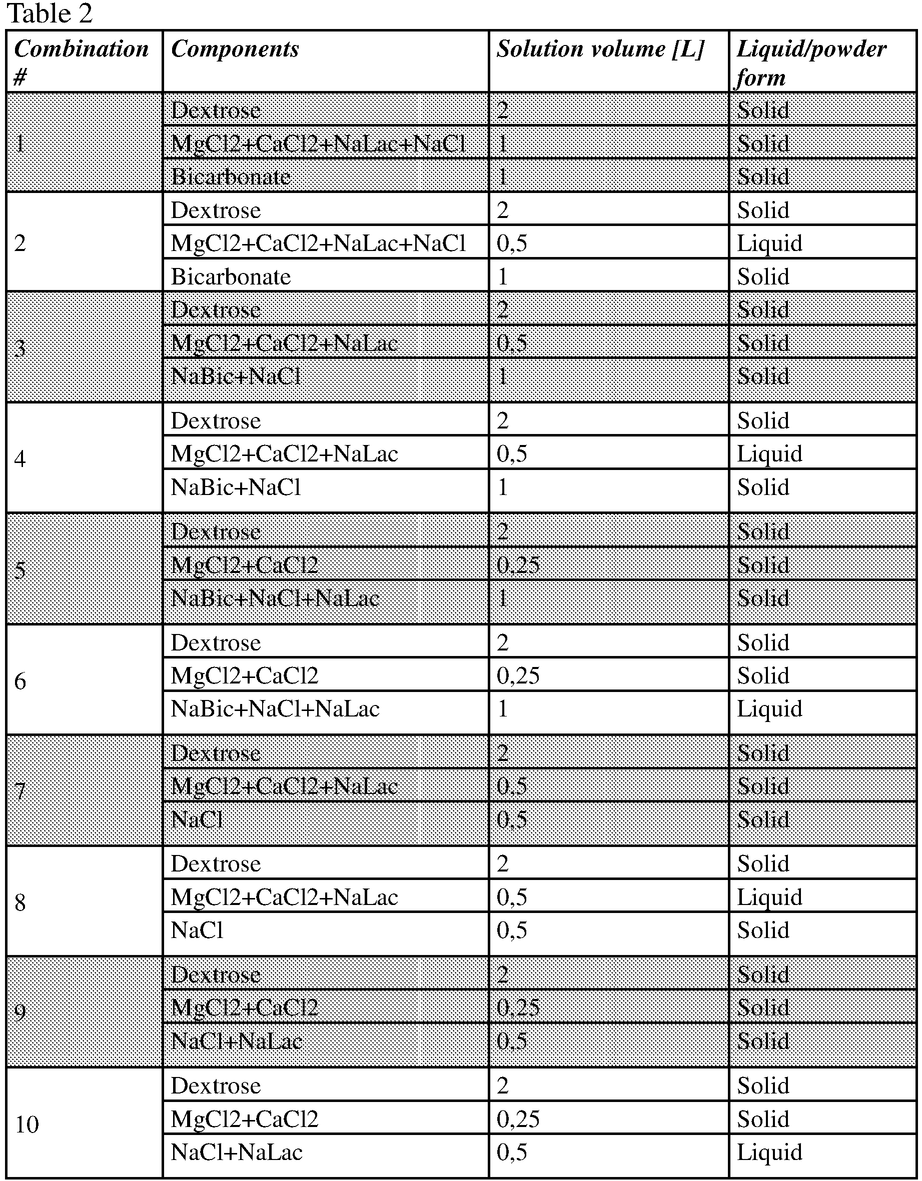

The peritoneal dialysis fluid generated using the concentrate pouches can include sodium chloride, sodium lactate, magnesium chloride, calcium chloride, dextrose, and optionally sodium bicarbonate. However, in certain embodiments, the lactate can be eliminated and only sodium bicarbonate used, or the sodium bicarbonate eliminated and only sodium lactate. In each of the combinations listed in Table 2, dextrose is provided in a concentrate pouch without any other components. Providing dextrose without additional components allows for the dextrose concentration of the resulting peritoneal dialysis fluid to be set independently of the sodium, magnesium, calcium, or buffer concentrations, allowing therapy to be customized based on the medical prescription. However, in certain embodiments, the dextrose can be included in a single concentrate pouch with one or more additional components. Other osmotic agents can be used in addition to, or in place of dextrose. For example, icodextrin or other polydextrin can be used as an osmotic agent for generating peritoneal dialysis fluid.

In certain embodiments, the magnesium chloride and calcium chloride can be provided separately from the sodium bicarbonate. Bicarbonate can be separated from the magnesium and calcium to avoid precipitation. While bicarbonate can be separated from the magnesium and calcium, any 2 or more concentrate containers can be used with the other components in any arrangement. For example, in combination 7 of Table 2, the magnesium and calcium ions are included with sodium lactate. In combination 5 of Table 2, the sodium lactate is separated from the magnesium and calcium. Sodium chloride can be included in any concentrate pouch. When both bicarbonate and lactate are used the bicarbonate and lactate may be in the same concentrate pouch, as shown in combination 5, but the bicarbonate can remain separate from the magnesium and calcium. Each of the components can be provided in either solid or liquid form. The actual combination used can depend on the needs of the user.

The claimed invention is defined by the features of independent claims 1 and 17. One skilled in the art will understand that various combinations and/or modifications and variations can be made in the described systems and methods depending upon the specific needs for operation. Various aspects disclosed herein may be combined in different combinations than the combinations specifically presented in the description and accompanying drawings. Moreover, features illustrated or described as being part of an aspect of the disclosure may be used in the aspect of the disclosure, either alone or in combination, or follow a preferred arrangement of one or more of the described elements. Depending on the example, certain acts or events of any of the processes or methods described herein may be performed in a different sequence, may be added, merged, or left out altogether (e.g., certain described acts or events may not be necessary to carry out the techniques). In addition, while certain aspects of this disclosure are described as performed by a single module or unit for purposes of clarity, the techniques of this disclosure may be performed by a combination of units or modules associated with, for example, a medical device.

## Claims

1. A concentrate pouch (100) for peritoneal dialysis systems, comprising:
a fluid inlet **characterized by** the fluid inlet being positioned at a bottom portion of the concentrate pouch (100) wherein the fluid inlet comprises a venturi tube (102) entering an interior (106) of the concentrate pouch (100) and extending upwardly into the interior of the concentrate pouch;
the venturi tube (102) comprising:
a venturi section, the venturi section being positioned in the interior of the concentrate pouch;
a first section distal to the interior portion of the concentrate pouch (100), the first section having a first diameter,
a second section distal to the first section and proximal to the interior of the concentrate pouch (100), the second section having a second diameter smaller than the first diameter forming a constriction (107), and
a third section distal to the second section and extending inwardly into the interior of the concentrate pouch, the third section having a third diameter larger than the second diameter, and having a suction port (103) at or about a floor of the interior of the concentrate pouch (100), the suction port (103) exposing an interior of the venturi tube (102) to the interior of the concentrate pouch, wherein the first diameter of the first section or the third diameter of the third section is about four times the second diameter of the second section.

2. The concentrate pouch of claim 1, wherein the concentrate pouch (100) contains a solid material.

3. The concentrate pouch of claim 1, wherein the concentrate pouch (100) is constructed of a flexible material.

4. The concentrate pouch of claim 1, wherein the concentrate pouch (100) is constructed of a flexible material and a stand up pouch.

5. The concentrate pouch of claim 1, wherein the floor of the concentrate pouch (100) inwardly tapers towards the fluid inlet.

6. The concentrate pouch of claim 1, the venturi tube (102) extending upwardly from a bottom of the concentrate pouch (100) towards a top of the concentrate pouch.

7. A system comprising one or more concentrate pouches (100) of claim 1, fluidly connected to a peritoneal dialysis fluid generation system; and
a control system; the control system programmed to pump water into the one or more concentrate pouches to generate a concentrate; and to pump a concentrate from the one or more concentrate pouches into the peritoneal dialysis fluid generation flow path and into a mixing container.

8. The system of claim 7, further comprising a water source fluidly connectable to the one or more concentrate pouches (100).

9. The system of claim 7, wherein the one or more concentrate pouches (100) comprise a concentrate pouch containing dextrose, icodextrin, or a polydextrin, a concentrate pouch containing magnesium, calcium and sodium lactate, and a concentrate pouch containing sodium bicarbonate and sodium chloride.

10. The system of claim 7, wherein the one or more concentrate pouches (100) comprise a concentrate pouch containing dextrose, icodextrin, or a polydextrin, a concentrate pouch containing magnesium and calcium, and a concentrate pouch containing sodium bicarbonate, sodium chloride, and sodium lactate.

11. The system of claim 7, wherein the one or more concentrate pouches (100) comprise a concentrate pouch containing dextrose, icodextrin, or a polydextrin, a concentrate pouch containing magnesium, calcium, and sodium lactate, and a concentrate pouch containing sodium chloride.

12. The system of claim 7, wherein the one or more concentrate pouches (100) comprise a concentrate pouch containing dextrose, icodextrin, or a polydextrin, a concentrate pouch containing magnesium and calcium, and a concentrate pouch containing sodium chloride and sodium lactate.

13. The system of any of claims 7-12, wherein at least one of the concentrate pouches (100) initially contains a solid substance.

14. The system of any of claims 7-12, wherein at least one of the concentrate pouches (100) initially contains a liquid or aqueous substance.

15. The system of any of claims 7-13, wherein the control system is programmed to pump an amount of water into the one or more concentrate pouches (100) to completely dissolve materials within the one or more concentrate pouches.

16. The system of any claims 7-13, wherein at least one of the one or more concentrate pouches (100) is a stand up pouch.

17. A method, comprising the steps of:
pumping water into one or more concentrate pouches (100) of claim 1;
dissolving a material within the one or more concentrate pouches to generate a concentrate in each of the one or more concentrate pouches; and
pumping the concentrate out of each of the one or more concentrate pouches (100).

18. The method of claim 17, further comprising the step of pumping the concentrate from each concentrate pouch (100) to a mixing container.

19. The method of claim 17, wherein the one or more concentrate pouches comprise a concentrate pouch (100) containing dextrose, icodextrin, or a polydextrin, a concentrate pouch containing magnesium, calcium and sodium lactate, and a concentrate pouch containing sodium bicarbonate and sodium chloride.

20. The method of claim 17, wherein the one or more concentrate pouches (100) comprise a concentrate pouch containing dextrose, icodextrin, or a polydextrin, a concentrate pouch containing magnesium and calcium, and a concentrate pouch containing sodium bicarbonate, sodium chloride, and sodium lactate.

21. The method of claim 17, wherein the one or more concentrate pouches(100) comprise a concentrate pouch containing dextrose, icodextrin, or a polydextrin, a concentrate pouch containing magnesium, calcium, and sodium lactate, and a concentrate pouch containing sodium chloride.

22. The method of claim 17, wherein the one or more concentrate pouches (100) comprise a concentrate pouch containing dextrose, icodextrin, or polydextrin, a concentrate pouch containing magnesium and calcium, and a concentrate pouch containing sodium chloride and sodium lactate.

## Patentansprüche

1. Konzentratbeutel (100) für Peritonealdialysesysteme, umfassend:
einen Fluideinlass, **dadurch gekennzeichnet, dass** der Fluideinlass an einem unteren Teil des Konzentratbeutels (100) positioniert ist, wobei der Fluideinlass ein Venturirohr (102), das in ein Inneres (106) des Konzentratbeutels (100) eintritt und sich nach oben in das Innere des Konzentratbeutels erstreckt, umfasst;
das Venturirohr (102) umfassend:
einen Venturiabschnitt, wobei der Venturiabschnitt in dem Inneren des Konzentratbeutels positioniert ist;
einen ersten Abschnitt distal zu dem inneren Teil des Konzentratbeutels (100), wobei der erste Abschnitt einen ersten Durchmesser aufweist,
einen zweiten Abschnitt distal zu dem ersten Abschnitt und proximal zu dem Inneren des Konzentratbeutels (100), wobei der zweite Abschnitt einen zweiten Durchmesser, der kleiner als der erste Durchmesser ist, aufweist, der eine Verengung (107) ausbildet, und
einen dritten Abschnitt distal zu dem zweiten Abschnitt und der sich nach innen in das Innere des Konzentratbeutels erstreckt, wobei der dritte Abschnitt einen dritten Durchmesser, der größer als der zweite Durchmesser ist, aufweist und eine Saugöffnung (103) an oder etwa an einem Boden des Inneren des Konzentratbeutels (100) aufweist, wobei die Saugöffnung (103) ein Inneres des Venturirohrs (102) dem Inneren des Konzentratbeutels aussetzt, wobei der erste Durchmesser des ersten Abschnitts oder der dritte Durchmesser des dritten Abschnitts etwa ein Vierfaches des zweiten Durchmesser des zweiten Abschnitts beträgt.

2. Konzentratbeutel nach Anspruch 1, wobei der Konzentratbeutel (100) ein festes Material enthält.

3. Konzentratbeutel nach Anspruch 1, wobei der Konzentratbeutel (100) aus einem flexiblen Material konstruiert ist.

4. Konzentratbeutel nach Anspruch 1, wobei der Konzentratbeutel (100) aus einem flexiblen Material konstruiert und ein Standbeutel ist.

5. Konzentratbeutel nach Anspruch 1, wobei sich der Boden des Konzentratbeutels (100) nach innen zu dem Fluideinlass hin verjüngt.

6. Konzentratbeutel nach Anspruch 1, wobei sich das Venturirohr (102) von einer Unterseite des Konzentratbeutels (100) nach oben zu einer Oberseite des Konzentratbeutels erstreckt.

7. System, umfassend einen oder mehrere Konzentratbeutel (100) nach Anspruch 1, die mit einem Peritonealdialysefluiderzeugungssystem fluidisch verbunden sind; und
ein Steuersystem; wobei das Steuersystem programmiert ist, um Wasser in den einen oder die mehreren Konzentratbeutel zu pumpen, um ein Konzentrat zu erzeugen; und um ein Konzentrat von dem einen oder den mehreren Konzentratbeuteln in den Peritonealdialysefluiderzeugungsströmungsweg und in einen Mischbehälter zu pumpen.

8. System nach Anspruch 7, ferner umfassend eine Wasserquelle, die mit dem einen oder den mehreren Konzentratbeuteln (100) fluidisch verbindbar ist.

9. System nach Anspruch 7, wobei der eine oder die mehreren Konzentratbeutel (100) einen Konzentratbeutel, der Dextrose, Icodextrin oder ein Polydextrin enthält, einen Konzentratbeutel, der Magnesium, Calcium und Natriumlactat enthält, und einen Konzentratbeutel, der Natriumbicarbonat und Natriumchlorid enthält, umfassen.

10. System nach Anspruch 7, wobei der eine oder die mehreren Konzentratbeutel (100) einen Konzentratbeutel, der Dextrose, Icodextrin oder ein Polydextrin enthält, einen Konzentratbeutel, der Magnesium und Calcium enthält, und einen Konzentratbeutel, der Natriumbicarbonat, Natriumchlorid und Natriumlactat enthält, umfassen.

11. System nach Anspruch 7, wobei der eine oder die mehreren Konzentratbeutel (100) einen Konzentratbeutel, der Dextrose, Icodextrin oder ein Polydextrin enthält, einen Konzentratbeutel, der Magnesium, Calcium und Natriumlactat enthält, und einen Konzentratbeutel, der Natriumchlorid enthält, umfassen.

12. System nach Anspruch 7, wobei der eine oder die mehreren Konzentratbeutel (100) einen Konzentratbeutel, der Dextrose, Icodextrin oder ein Polydextrin enthält, einen Konzentratbeutel, der Magnesium und Calcium enthält, und einen Konzentratbeutel, der Natriumchlorid und Natriumlactat enthält, umfassen.

13. System nach einem der Ansprüche 7 bis 12, wobei mindestens einer der Konzentratbeutel (100) anfänglich eine feste Substanz enthält.

14. System nach einem der Ansprüche 7 bis 12, wobei mindestens einer der Konzentratbeutel (100) anfänglich eine flüssige oder wässrige Substanz enthält.

15. System nach einem der Ansprüche 7 bis 13, wobei das Steuersystem programmiert ist, um eine Wassermenge in den einen oder die mehreren Konzentratbeutel (100) zu pumpen, um Materialien innerhalb des einen oder der mehreren Konzentratbeutel vollständig aufzulösen.

16. System nach einem der Ansprüche 7 bis 13, wobei mindestens einer des einen oder der mehreren Konzentratbeutel (100) ein Standbeutel ist.

17. Verfahren, umfassend die Schritte:
Pumpen von Wasser in einen oder mehrere Konzentratbeutel (100) nach Anspruch 1;
Auflösen eines Materials innerhalb des einen oder der mehreren Konzentratbeutel, um ein Konzentrat in jedem des einen oder der mehreren Konzentratbeutel zu erzeugen; und
Pumpen des Konzentrats aus jedem des einen oder der mehreren Konzentratbeutel (100).

18. Verfahren nach Anspruch 17, ferner umfassend den Schritt des Pumpens des Konzentrats von jedem Konzentratbeutel (100) zu einem Mischbehälter.

19. Verfahren nach Anspruch 17, wobei der eine oder die mehreren Konzentratbeutel einen Konzentratbeutel (100), der Dextrose, Icodextrin oder ein Polydextrin enthält, einen Konzentratbeutel, der Magnesium, Calcium und Natriumlactat enthält, und einen Konzentratbeutel, der Natriumbicarbonat und Natriumchlorid enthält, umfassen.

20. Verfahren nach Anspruch 17, wobei der eine oder die mehreren Konzentratbeutel (100) einen Konzentratbeutel, der Dextrose, Icodextrin oder ein Polydextrin enthält, einen Konzentratbeutel, der Magnesium und Calcium enthält, und einen Konzentratbeutel, der Natriumbicarbonat, Natriumchlorid und Natriumlactat enthält, umfassen.

21. Verfahren nach Anspruch 17, wobei der eine oder die mehreren Konzentratbeutel (100) einen Konzentratbeutel, der Dextrose, Icodextrin oder ein Polydextrin enthält, einen Konzentratbeutel, der Magnesium, Calcium und Natriumlactat enthält, und einen Konzentratbeutel, der Natriumchlorid enthält, umfassen.

22. Verfahren nach Anspruch 17, wobei der eine oder die mehreren Konzentratbeutel (100) einen Konzentratbeutel, der Dextrose, Icodextrin oder Polydextrin enthält, einen Konzentratbeutel, der Magnesium und Calcium enthält, und einen Konzentratbeutel, der Natriumchlorid und Natriumlactat enthält, umfassen.

## Revendications

1. Sachet de concentré (100) pour systèmes de dialyse péritonéale, comprenant :
une entrée de fluide **caractérisée par** l'entrée de fluide étant positionnée au niveau d'une partie inférieure du sachet de concentré (100), dans lequel l'entrée de fluide comprend un tube venturi (102) entrant un intérieur (106) du sachet de concentré (100) et s'étendant vers le haut à l'intérieur du sachet de concentré ;
le tube venturi (102) comprenant :
une section venturi, la section venturi étant positionnée dans l'intérieur du sachet de concentré ;
une première section distale par rapport à la partie intérieure du sachet de concentré (100), la première section ayant un premier diamètre,
une deuxième section distale par rapport à la première section et proximale par rapport à l'intérieur du sachet de concentré (100), la deuxième section ayant un deuxième diamètre inférieur au premier diamètre formant une constriction (107), et
une troisième section distale par rapport à la deuxième section et s'étendant vers l'intérieur du sachet de concentré, la troisième section ayant un troisième diamètre plus grand que le deuxième diamètre, et ayant un orifice d'aspiration (103) au niveau ou autour d'un fond de l'intérieur du sachet de concentré (100), l'orifice d'aspiration (103) exposant un intérieur du tube venturi (102) à l'intérieur du sachet de concentré, dans lequel le premier diamètre de la première section ou le troisième diamètre de la troisième section est d'environ quatre fois le deuxième diamètre de la deuxième section.

2. Sachet de concentré selon la revendication 1, dans lequel le sachet de concentré (100) contient un matériau solide.

3. Sachet de concentré selon la revendication 1, dans lequel le sachet de concentré (100) est constitué d'un matériau flexible.

4. Sachet de concentré selon la revendication 1, dans lequel le sachet de concentré (100) est constitué d'un matériau flexible et d'un sachet à fond plat.

5. Sachet de concentré selon la revendication 1, dans lequel le fond du sachet de concentré (100) se rétrécit vers l'intérieur en direction de l'entrée de fluide.

6. Sachet de concentré selon la revendication 1, le tube venturi (102) s'étendant vers le haut à partir d'une partie inférieure du sachet de concentré (100) vers une partie supérieure du sachet de concentré.

7. Système comprenant un ou plusieurs sachets de concentré (100) selon la revendication 1, reliés fluidiquement à un système de génération de fluide de dialyse péritonéale ; et
un système de commande ; le système de commande programmé pour pomper de l'eau dans le ou les sachets de concentré afin de générer un concentré ; et pour pomper un concentré à partir du ou des sachets de concentré dans le circuit de génération du liquide de dialyse péritonéale et dans un récipient de mélange.

8. Système selon la revendication 7, comprenant en outre une source d'eau pouvant être raccordée fluidiquement au ou aux sachets de concentré (100).

9. Système selon la revendication 7, dans lequel le ou les sachets de concentré (100) comprennent un sachet de concentré contenant du dextrose, de l'icodextrine ou une polydextrine, un sachet de concentré contenant du magnésium, du calcium et du lactate de sodium, et un sachet de concentré contenant du bicarbonate de sodium et du chlorure de sodium.

10. Système selon la revendication 7, dans lequel le ou les sachets de concentré (100) comprennent un sachet de concentré contenant du dextrose, de l'icodextrine ou une polydextrine, un sachet de concentré contenant du magnésium et du calcium, et un sachet de concentré contenant du bicarbonate de sodium, du chlorure de sodium et du lactate de sodium.

11. Système selon la revendication 7, dans lequel le ou les sachets de concentré (100) comprennent un sachet de concentré contenant du dextrose, de l'icodextrine ou une polydextrine, un sachet de concentré contenant du magnésium, du calcium et du lactate de sodium, et un sachet de concentré contenant du chlorure de sodium.

12. Système selon la revendication 7, dans lequel le ou les sachets de concentré (100) comprennent un sachet de concentré contenant du dextrose, de l'icodextrine ou une polydextrine, un sachet de concentré contenant du magnésium et du calcium, et un sachet de concentré contenant du chlorure de sodium et du lactate de sodium.

13. Système selon l'une quelconque des revendications 7-12, dans lequel au moins l'un des sachets de concentré (100) contient initialement une substance solide.

14. Système selon l'une quelconque des revendications 7-12, dans lequel au moins l'un des sachets de concentré (100) contient initialement une substance liquide ou aqueuse.

15. Système selon l'une quelconque des revendications 7-13, dans lequel le système de commande est programmé pour pomper une quantité d'eau dans le ou les sachets de concentré (100) afin de dissoudre complètement les matériaux dans le ou les sachets de concentré.

16. Système selon l'une quelconque des revendications 7-13, dans lequel au moins l'un parmi le ou les sachets de concentré (100) est un sachet à fond plat.

17. Procédé, comprenant les étapes consistant à :
pomper de l'eau dans un ou plusieurs sachets de concentré (100) selon la revendication 1 ;
dissoudre un matériau dans le ou les sachets de concentré pour générer un concentré dans chacun parmi le ou les sachets de concentré ; et
pomper le concentré hors de chacun parmi le ou les sachets de concentré (100).

18. Procédé selon la revendication 17, comprenant en outre l'étape consistant à pomper le concentré de chaque sachet de concentré (100) vers un réservoir de mélange.

19. Procédé selon la revendication 17, dans lequel le ou les sachets de concentré comprennent un sachet de concentré (100) contenant du dextrose, de l'icodextrine ou une polydextrine, un sachet de concentré contenant du magnésium, du calcium et du lactate de sodium, et un sachet de concentré contenant du bicarbonate de sodium et du chlorure de sodium.

20. Procédé selon la revendication 17, dans lequel le ou les sachets de concentré (100) comprennent un sachet de concentré contenant du dextrose, de l'icodextrine ou une polydextrine, un sachet de concentré contenant du magnésium et du calcium, et un sachet de concentré contenant du bicarbonate de sodium, du chlorure de sodium et du lactate de sodium.

21. Procédé selon la revendication 17, dans lequel le ou les sachets de concentré (100) comprennent un sachet de concentré contenant du dextrose, de l'icodextrine ou une polydextrine, un sachet de concentré contenant du magnésium, du calcium et du lactate de sodium, et un sachet de concentré contenant du chlorure de sodium.

22. Procédé selon la revendication 17, dans lequel le ou les sachets de concentré (100) comprennent un sachet de concentré contenant du dextrose, de l'icodextrine ou de la polydextrine, un sachet de concentré contenant du magnésium et du calcium, et un sachet de concentré contenant du chlorure de sodium et du lactate de sodium.
